# EUROPEAN PATENT APPLICATION

(11) **EP 3 216 814 A1**
(43) Date of publication of application: **13.09.2017**
(21) Application number: 16173252.4
(22) Date of filing: 07.06.2016
(51) Int. Cl.: C08G 59/26, C08G 59/14

(54) **ISOCYANATE-MODIFIED EPOXY RESIN AND USES THEREOF**

(30) Priority: 08.03.2016 CN 201610129829
(71) Applicant: Guangdong Guangshan New Materials Co., Ltd., Guangdong 523000 (CN)
(72) Inventor: PAN, Qingchong, 523000 Dongguan (CN)
(74) Representative: Brevalex

(57) **Abstract**

The present invention relates to an isocyanate-modified epoxy resin having a structure of Formula (I): in Formula (I), R is selected from divalent organic groups; n is an integer greater than or equal to zero; in Y, R₁, R₂, R₃, R₄, R₁₈, R₁₉, R₂₀, R₂₁ are independently selected from organic groups; X is nothingness or is selected from divalent organic groups; and R₁, R₂, R₃, R₄, R₁₈, R₁₉, R₂₀, R₂₁ are not hydrogen atoms at the same time. The epoxy resin of the present invention utilizes polyisocyanate containing more than 2 cyanate groups as a raw material for preparation and reacts it with resin monomer having epoxy group, thereby achieving the purpose of introducing oxazolidinonyl into epoxy resin monomer; in addition, the epoxy resin of the present invention is terminated by epoxy groups, making the provided epoxy resin components have high heat resistance and low dielectric properties.

## Description

### Technical field

The present invention belongs to the field of preparation of an epoxy resin, and relates to an epoxy resin and uses thereof.

### Background art

Epoxy resins generally refer to organic compounds containing two or more epoxy groups in molecular structures thereof, and their relative molecular masses are not high with a few exceptions. The molecular structure of an epoxy resin is characterized by active epoxy groups contained in the molecular chain thereof, and the epoxy groups can locate at the end or the middle of the molecular chain, or form a cyclic structure. Due to the active epoxy groups in the molecular structure, the epoxy resin can react with various types of curing agents by crosslinking to form an insoluble high polymer with three-dimensional network structure. All the polymers having epoxy groups in their molecule structures are collectively referred as epoxy resins. A cured epoxy resin has good physical and chemical properties, and excellent adhesion strength to the surface of metal and non-metallic materials, good dielectric properties, low variable shrinkage rate, good dimensional stability for products thereof, high hardness, good flexibility, and stability on alkali and most solvents, and thus it is widely used in various sectors of national defense and national economy for pouring, dipping or as laminated materials, adhesives, coatings, etc.

For the purpose of safety, electronic products represented by mobile phone, computer, video camera and electronic game machine, household and office electrical products represented by air conditioners, refrigerators, television images, audio products etc., and various products used in other areas largely require low dielectric properties and heat resistance.

In terms of properties of electrical appliances, the main factors to be considered also include the dielectric constant and dielectric loss of materials thereof. Generally speaking, because the signal transmission speed of the substrate is inversely proportional to the square root of the dielectric constant of the substrate material, it is generally better that the dielectric constant of the substrate material is smaller; on the other hand, since smaller dielectric loss represents less loss of signal transmission, the transmission quality provided by the material with smaller dielectric loss is better.

Therefore, a problem urgently to be solved in the area of printed circuit board materials at the present stage is how to develop materials with low dielectric constant and low dielectric loss and to apply them to the preparation of high frequency printed circuit boards.

### Contents of the invention

The first purpose of the present invention is to provide an isocyanate-modified epoxy resin having a structure of Formula (I): 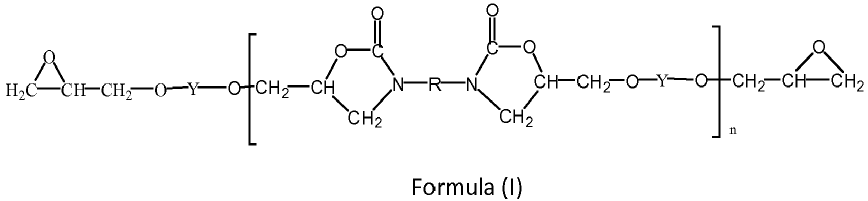 wherein,
R is selected from divalent organic groups;
n is an integer greater than or equal to zero;
Y is selected from 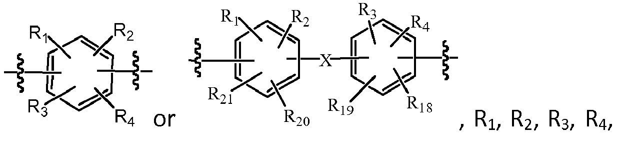 R₁₈, R₁₉. R₂₀, R₂₁ are independently selected from organic groups; X is nothingness or is selected from divalent organic groups; and R₁, R₂, R₃, R₄, R₁₈, R₁₉, R₂₀, R₂₁ are not hydrogen atoms at the same time.

As one of preferred embodiments, the epoxy resin has a structure of Formula (II): 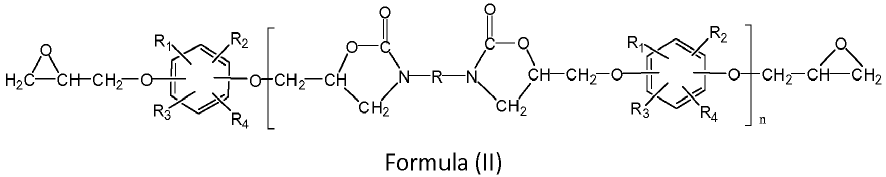 wherein,
R is selected from divalent organic groups;
R₁, R₂, R₃, R₄ are independently selected from organic groups, and R₁, R₂, R₃, R₄ are not hydrogen atoms at the same time;
n is an integer greater than or equal to zero, e.g., 1, 2, 3, 4, 5, ,6, 7, 8, 9, 10 etc.

As a second preferred embodiment, the epoxy resin has a structure of Formula (III): 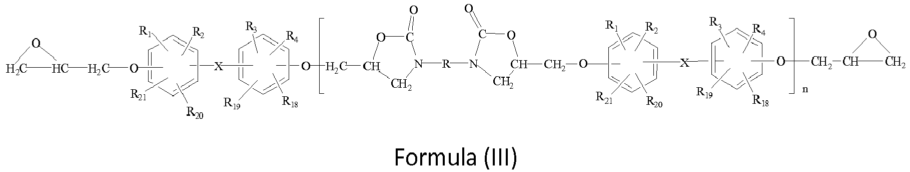 wherein,
X is nothingness or is selected from divalent organic groups;
R is selected from divalent organic groups;
R₁, R₂, R₃, R₄, R₁₈, R₁₉, R₂₀, R₂₁ are independently selected from organic groups, and R₁, R₂, R₃, R₄, R₁₉, R₁₉, R₂₀, R₂₁ are not hydrogen atoms at the same time;
n is an integer greater than or equal to zero.

Preferably, the epoxy resin has a structure of Formula (IV): 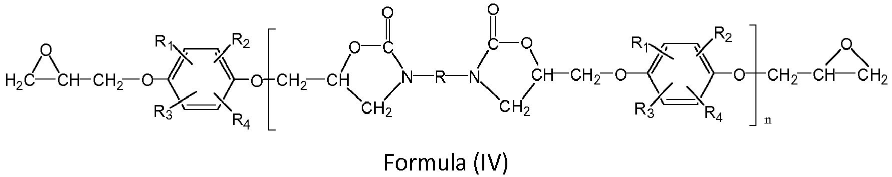 wherein,
R is selected from divalent organic groups;
R₁, R₂, R₃, R₄ are independently selected from organic groups, and R₁, R₂, R₃, R₄ are not hydrogen atoms at the same time;
n is an integer greater than or equal to zero.

Or preferably, the epoxy resin has a structure of Formula (V): 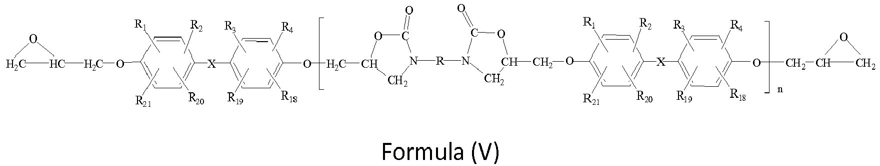 wherein,
X, R are selected from divalent organic groups;
R₁, R₂, R₃, R₄, R₁₈, R₁₉, R₂₀, R₂₁ are independently selected from organic groups, and R₁, R₂, R₃, R₄, R₁₉, R₁₉, R₂₀, R₂₁ are not hydrogen atoms at the same time;
n is an integer greater than or equal to zero.

Preferably, R is selected from substituted or unsubstituted straight chain alkylene, substituted or unsubstituted branched alkylene, substituted or unsubstituted arylene/arylidene; preferably C₁-C₃₀ substituted or unsubstituted straight chain alkylene, C₁-C₃₀ substituted or unsubstituted branched alkylene, C₆-C₃₀ substituted or unsubstituted arylene/arylidene; further preferably 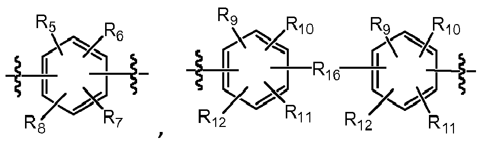 or 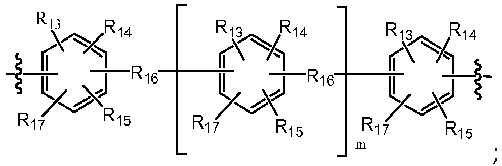 wherein,
R₅, R₆, R₇, R₈, R₉, R₁₀, R₁₁, R₁₂, R₁₃, R₁₄, R₁₅, R₁₇ are independently selected from organic groups, preferably selected from H, substituted or unsubstituted straight chain hydrocarbyl, substituted or unsubstituted branched hydrocarbyl, or substituted or unsubstituted aryl; preferably C₁-C₃₀ substituted or unsubstituted straight chain hydrocarbyl, C₁-C₃₀ substituted or unsubstituted branched hydrocarbyl, or C₆-C₃₀ substituted or unsubstituted aryl;
R₁₆ is independently selected from organic groups, preferably selected from substituted or unsubstituted straight chain alkylene, substituted or unsubstituted branched alkylene, or substituted or unsubstituted arylene/arylidene; preferably C₁-C₃₀ substituted or unsubstituted straight chain alkylene, C₁-C₃₀ substituted or unsubstituted branched alkylene, or C₆-C₃₀ substituted or unsubstituted arylene/arylidene;
m is an integer greater than or equal to zero.

Further preferably, R₅, R₆, R₇, R₈, R₉, R₁₀, R₁₁, R₁₂ are H;
preferably, R₁₃, R₁₄, R₁₅, R₁₇ are independently selected from substituted or unsubstituted N-oxazolidinonyl;
preferably, R₁₆ is selected from C₁-C₅ substituted or unsubstituted straight chain alkylene, preferably any one of methylene, ethylidene/ethylene or n-propylidene/ n-propylene.

Preferably, X is nothingness or substituted or unsubstituted straight chain alkylene, substituted or unsubstituted branched alkylene, substituted or unsubstituted arylene/arylidene; preferably nothingness or C₁-C₃₀ substituted or unsubstituted straight chain alkylene, C₁-C₃₀ substituted or unsubstituted branched alkylene, C₆-C₃₀ substituted or unsubstituted arylene/arylidene; further preferably nothingness or any one of methylene, isopropylidene/isopropylene, isobutylidene/isobutylene, cyclohexylidene/cyclohexylene, carbonyl; most preferably nothingness or methylene or isopropylidene/isopropylene;
preferably, R is selected from 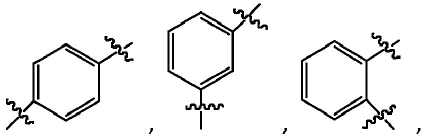 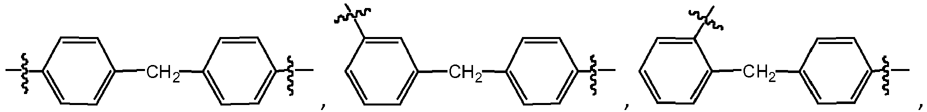 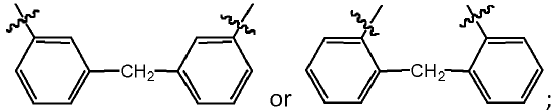 preferably, R₁, R₂, R₃, R₄, R₁₈, R₁₉, R₂₀, R₂₁ are independently selected from H, substituted or unsubstituted straight chain hydrocarbyl, substituted or unsubstituted branched hydrocarbyl, or substituted or unsubstituted aryl; preferably C₁-C₃₀ substituted or unsubstituted straight chain hydrocarbyl, C₁-C₃₀ substituted or unsubstituted branched hydrocarbyl, or C₆-C₃₀ substituted or unsubstituted aryl; preferably any one of methyl, ethyl, n-propyl, isopropyl, n-butyl or tert-butyl, or a combination of at least two of them.

The isocyanate-modified epoxy resin of the present invention includes, but is not limited to: 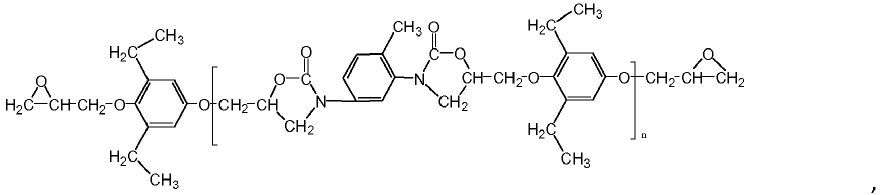 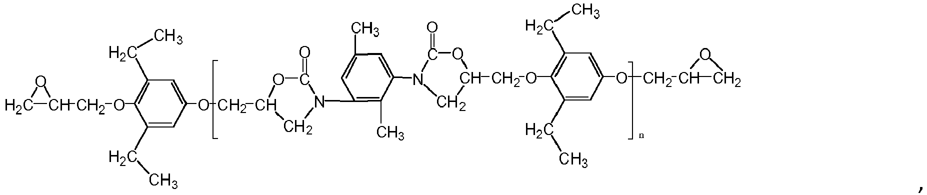 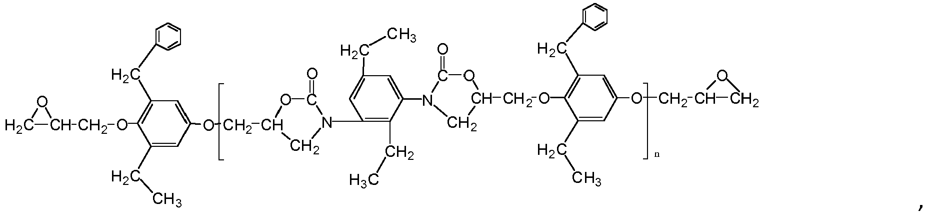 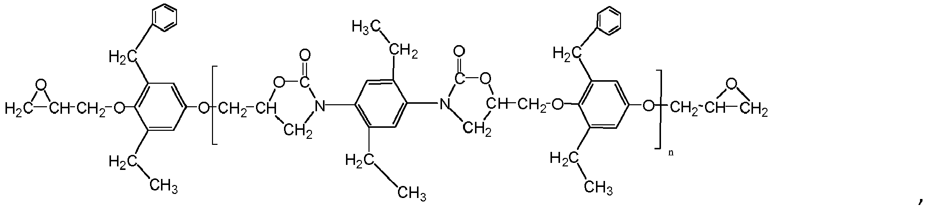 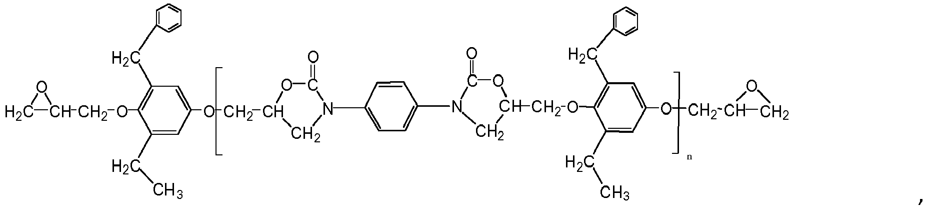 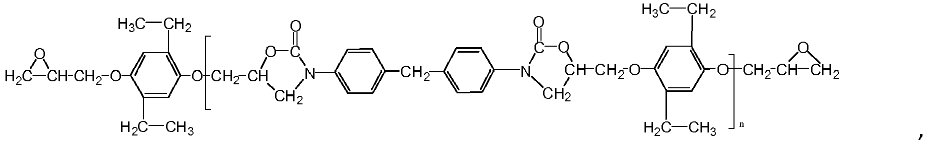 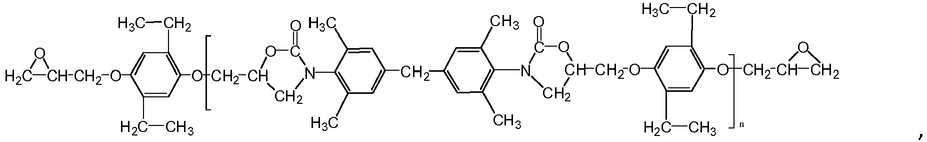 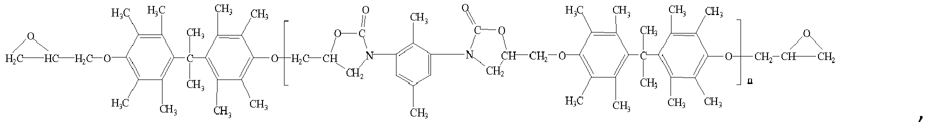 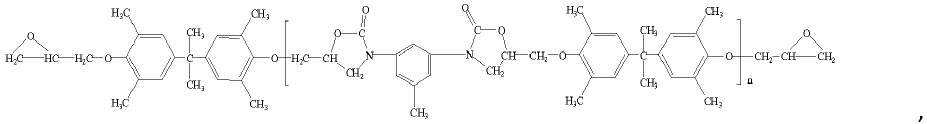 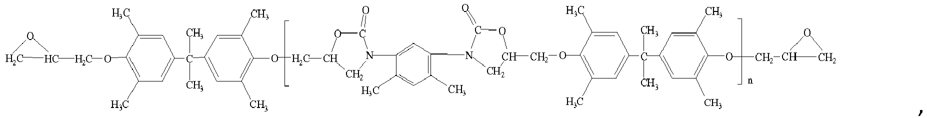 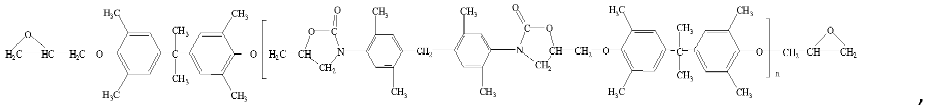 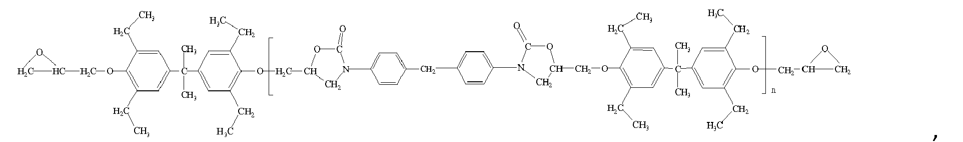 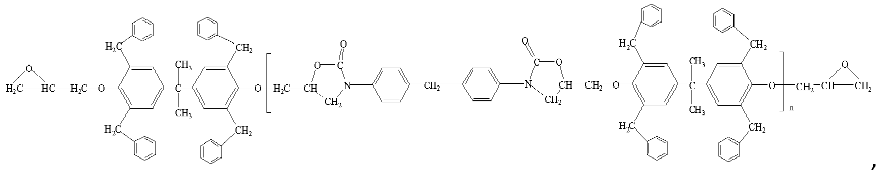 etc.

A typical method for preparing the isocyanate-modified epoxy resin of the present invention is: reacting polyisocyanate having at least 2 cyanate groups with a substance having bisphenol ether structure to obtain oxazolidinone ring.

The general structure of the substance having bisphenol ether structure is 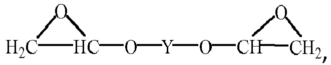 wherein, Y has options within the aforementioned ranges.

Typical but non-limiting examples include any one of bisphenol A epoxy ether, bisphenol F epoxy ether, bisphenol Z epoxy ether, or a combination of at least two of them.

As a non-limiting example, the preparation method of the isocyanate-modified epoxy resin comprises the following steps:
(1) the substance having bisphenol ether structure was put into a reaction kettle, and heated, preferably heated to a temperature of 60-190°C, more preferably to a temperature of 120-160°C;
(2) a polyisocyanate having at least 2 cyanate groups was added, and the mixture was stirred;
(3) known catalysts such as imidazoles, amines and salts thereof, and triphenylphosphine and derivatives thereof and so on were added, and the isocyanate-modified epoxy resin of the present invention was thereby obtained after the reaction was completed.

The second purpose of the present invention is to provide an epoxy resin composition comprising an epoxy resin and a curing agent; wherein a part or all of the epoxy resin is the isocyanate-modified epoxy resin described in the first purpose of the invention;
preferably, the epoxy resin also comprises any one of liquid bisphenol A epoxy resin, liquid bisphenol F epoxy resin, solid bisphenol A epoxy resin, solid bisphenol F epoxy resin, bisphenol S epoxy resin, cyclopentadiene epoxy resin or diphenyl epoxy resin, or a combination of at least two of them;
preferably, the curing agent also comprises any one of amines, polyphenol compounds, ester compounds, acids and anhydrides, or a combination of at least two of them;
preferably, the epoxy resin composition also comprises a curing accelerator, and the curing accelerator is any one of imidazole curing accelerators, organophosphine curing accelerators, tertiary amine curing accelerators, or pyridines and derivatives thereof, or a mixture of at least two of them;
preferably, the epoxy resin composition also comprises an inorganic filler;
preferably, the epoxy resin composition also comprises a flame retardant;
preferably, the epoxy resin composition also comprises a mold discharging agent.

The third purpose of the present invention is to provide a prepreg prepared by impregnating a substrate with the epoxy resin composition of the second purpose or coating the epoxy resin composition onto a substrate;
preferably, the substrate is a glass fiber substrate, a polyester substrate, a polyimide substrate, a ceramic substrate or a carbon fiber substrate.

The fourth purpose of the present invention is to provide a composite metal laminate comprising more than one sheet of the prepreg as described in the third purpose and prepared by coating metal layer on the surface of the prepregs, overlapping and pressing successively;
preferably, the material of the surface-coated metal layer is aluminium, copper, iron and alloys of any combination thereof;
preferably, the composite metal laminate is CEM-1 copper clad laminate, CEM-3 copper clad laminate, FR-4 copper clad laminate, FR-5 copper clad laminate, CEM-1 aluminum clad laminate, CEM-3 aluminum clad laminate, FR-4 aluminum clad laminate or FR-5 aluminum clad laminate.

The fifth purpose of the present invention is to provide a wiring board prepared by processing wires on the surface of the composite metal laminate as described in the fourth purpose.

Compared with the prior arts, the present invention has the following beneficial effects:
The epoxy resin of the present invention utilizes polyisocyanate containing more than 2 cyanate groups as a raw material for preparation and reacts it with resin monomer having epoxy group, thereby achieving the purpose of introducing oxazolidinonyl into the epoxy resin monomer; in addition, the epoxy resin of the present invention is terminated by epoxy groups, making the provided epoxy resin components have high heat resistance and low dielectric properties.

### Embodiments

The technical solution of the present invention is further described by the following embodiments.

Those skilled in the art should appreciate that the examples are only used to facilitate understanding the present invention and shall not be deemed as limitations to the present invention.

### Example 1

Compound 1 has the following structure:

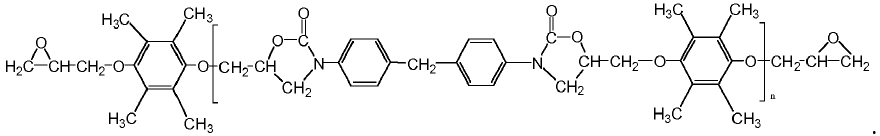

The preparation method is:
(1) 230 g (1.5eq) of epoxy resin with an epoxide equivalent of 153 g/eq having the following structure and 0.1 g of tetramethylammonium chloride were put into a 1000 mL three-neck glass reactor equipped with a stirring apparatus. The mixture was stirred while introducing nitrogen therein, and the temperature was raised to 105°C at the same time. Then 62.5 g of 4,4'-MDI (0.5eq) was dripped evenly taking 120 minutes. After that, the temperature was raised to 130 °C and the mixture continued to react for 640 minutes, and thereby 292.5 g of epoxy resin with epoxide equivalent of 320 g/eq was obtained. The product epoxy resin was named as epoxy resin A.

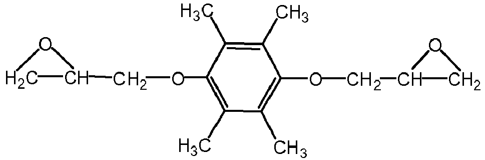

### Structure Characterizations:

Infrared Spectroscopy: epoxy group 913-916cm⁻¹; ether group 1230-1010cm⁻¹; oxazolidonyl group 1755cm⁻¹; methyl 2960 cm⁻¹, 2870 cm⁻¹;
Nuclear Magnetic Resonance 1H-NMR (DMSO-d6, ppm): 7.50-7.55(m, hydrogen on phenyl in N-Ar-C); 7.00-7.10(m, hydrogen on phenyl in N-Ar-C); 2.47-2.52(m, hydrogen on methylene in 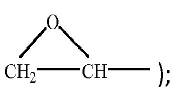 3.00-3.10(m, hydrogen on methyl in 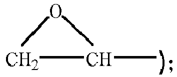 5.05-5.12 (hydrogen on methyl in oxazolidinonyl); 4.03-4.10 (hydrogen on CH₂ connected to epoxy group); 4.18 (hydrogen on CH₂ connected to oxazolidinonyl); 3.81(hydrogen on carbon connected to 2 benzene rings); 2.3-2.4 (hydrogen on methyl on benzene ring).

### Example 2

Compound 2 has the following structure: 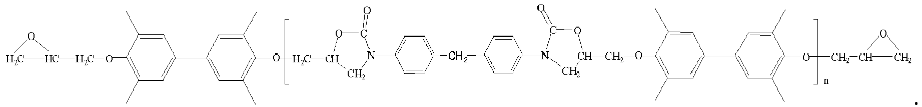 (1) 273 g (1.4eq) of epoxy resin with an epoxide equivalent of 195 g/eq having the following structure and 0.1 g of tetramethylammonium chloride were put into a 1000 mL three-neck glass reactor equipped with a stirring apparatus. The mixture was stirred while introducing nitrogen therein, and the temperature was raised to 105°C at the same time. Then 62.5 g of 4,4'-MDI (0.5eq) was dripped evenly taking 240 minutes. After that, the temperature was raised to 130 °C and the mixture continued to react for 640 minutes, and thereby 335.5 g of epoxy resin with epoxide equivalent of 373 g/eq was obtained. The product epoxy resin was named as epoxy resin B.

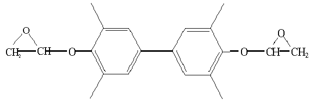

### Structure Characterizations:

Infrared Spectroscopy: epoxy group 913-916 cm⁻¹; ether group 1230-1010 cm⁻¹; oxazolidonyl group 1755 cm⁻¹;
n is 10 by measurement;

Nuclear Magnetic Resonance 1H-NMR (DMSO-d6, ppm): 7.0-7.1(hydrogen on phenyl in N-Ar-C); 7.50-7.55(hydrogen on phenyl in N-Ar-C); 6.95-7.02 (hydrogen on biphenyl); 2.47-2.52 (m, hydrogen on methylene in 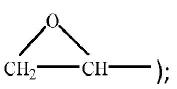 3.00-3.10 (m, hydrogen on methyl in 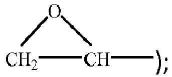 5.05-5.12 (hydrogen on methyl in oxazolidinonyl); 4.03-4.10 (hydrogen on CH₂ connected to epoxy group); 4.18 (hydrogen on CH₂ connected to oxazolidinonyl).

### Example 3

100 g of epoxy resin A obtained in Example 1, 25.0 g of linear phenolic resin with a phenolic hydroxyl equivalent of 105 g/eq and 0.1 g of 2-phenylimidazole were dissolved in appropriate acetone to form a solution. Standard glass fiber cloth was used for sizing. Then they were pressed into a copper clad laminate, which was named as copper clad laminate a. The measured properties of the copper clad laminate a are shown in Table 1.

### Example 4

100 g of epoxy resin B obtained in Example 2, 63.7 g of resin compound having the following structure with an ester equivalent of 220 and 0.2 g of pyridine were dissolved in appropriate acetone to form a solution. Standard glass fiber cloth was used for sizing. Then they were pressed into a copper clad laminate, which was named as copper clad laminate b. The measured properties of the copper clad laminate b are shown in Table 1. 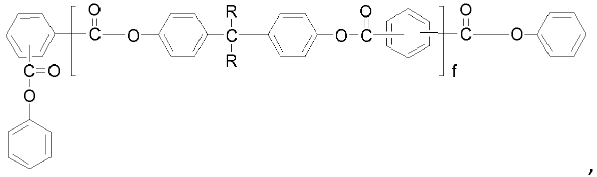 wherein, R is ethyl, f is 5. Comparative Example 1

100 g of commercially available MDI-modified epoxy resin with an epoxide equivalent of 380.0 g/eq, 27.3 g of linear phenolic resin with a phenolic hydroxyl equivalent of 105 g/eq and 0.1 g of 2-phenylimidazole were dissolved in appropriate acetone to form a solution. Standard glass fiber cloth was used for sizing. Then they were pressed into a copper clad laminate, which was named as copper clad laminate c. The measured properties of the copper clad laminate c are shown in Table 1.

### Comparative Example 2

100 g of commercially available MDI-modified epoxy resin with an epoxide equivalent of 380.0 g/eq, 57.9 g of resin compound having the following structure with an ester equivalent of 220 and 0.2 g of 2- pyridine were dissolved in appropriate acetone to form a solution. Standard glass fiber cloth was used for sizing. Then they were pressed into a copper clad laminate, which was named as copper clad laminate d. The measured properties of the copper clad laminate d are shown in Table 1. 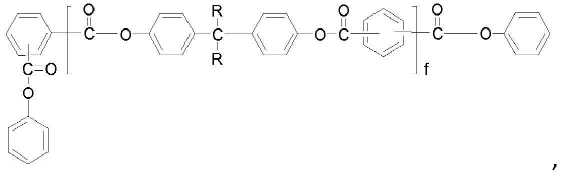 wherein, R is ethyl, f is 5.

### Performance tests

The following performance tests were carried out on copper clad laminate a, copper clad laminate b, copper clad laminate c and copper clad laminate d.

### (1) Dielectric constant/dielectric loss

A sample with 50mmx50mm size was prepared. A copper layer with a thickness of about 100 Å was vapor deposited on the surface of the sample. Then the sample was placed between two testing plate electrodes and the dielectric constant thereof in specified frequency range was tested.

### (2) Water absorption

A 100mm×100mm×1.6mm board was placed in an oven at 105 °C to dry for 1 h, and was weighted after cooling and then was steamed under a vapor pressure of 105kPa for 120 min, and finally was wiped and weighted, and then the water absorption thereof was calculated.

### (3) Glass transition temperature

A sample with a width of about 8-12 mm and a length of 60 mm was prepared and the glass transition temperature thereof was measured.

### (4) Bending strength and bending modulus

A 25mmx65mm sample was prepared, and the thickness thereof was measured using a vernier caliper, and the bending strength and bending modulus thereof were measured by adjusting the test mode of the universal material testing machine to bending test mode.

### (5) Tensile strength

A 250mmx25mm sample was prepared, and the thickness thereof was measured using a vernier caliper, and the tensile strength thereof was measured by adjusting the test mode of the universal material testing machine to tensile test mode.

### (6) Measurement of peeling strength

The copper-clad laminate was cut into a 100mmx3mm test piece. The peeling strength of copper foil and resin was measured by stripping the copper foil and delaminating it at a speed of 50.8 mm/min using peeling resistance test device.

The tested performance results of the copper foil substrate prepared by Examples are shown in Table 1.

**Table 1 Tested performance results of the copper foil substrates prepared by Examples**

| Items | copper clad laminate a | copper clad laminate b | copper clad laminate c | copper clad laminate d |
|---|---|---|---|---|
| Dielectric constant (5MHz) | 3.0 | 3.3 | 4.8 | 5.2 |
| Dielectric loss (5MHz) | 0.003 | 0.006 | 0.036 | 0.038 |
| Water absorption (%) | 0.38 | 0.35 | 0.65 | 0.74 |
| Tg (°C) | 170 | 158 | 136 | 132 |
| Peeling strength (N-mm⁻¹) | 2.03 | 2.05 | 1.46 | 1.52 |

### Example 5

Compound 3 has the following structure:

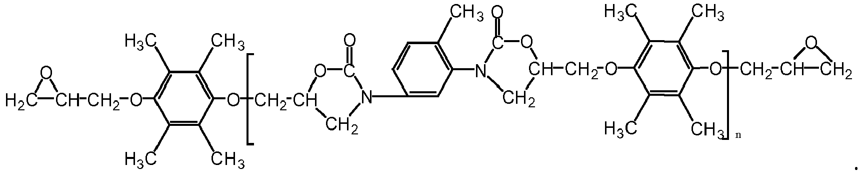

The preparation method is:
(1) 197.6 g (1.3eq) of epoxy resin with an epoxide equivalent of 152 g/eq having the following structure and 0.2 g of 2-methylimidazole were put into a 1000 mL three-neck glass reactor equipped with a stirring apparatus. The mixture was stirred while introducing nitrogen therein, and the temperature was raised to 105°C at the same time. Then 26.1 g of 2,4'-TDI (0.5eq) was dripped evenly taking 240 minutes. After that, the temperature was raised to 130 °C and the mixture continued to react for 640 minutes, and thereby 223.7 g of epoxy resin with epoxide equivalent of 230 g/eq was obtained. The product epoxy resin was named as epoxy resin C.

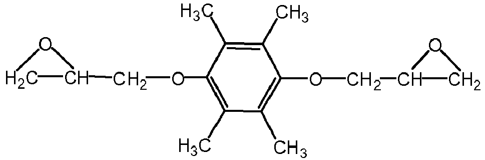

### Structure Characterizations:

Infrared Spectroscopy: epoxy group 913-916cm⁻¹; ether group 1230-1010cm⁻¹; oxazolidonyl group 1755cm⁻¹; methyl 2960 cm⁻¹, 2870 cm⁻¹;
Nuclear Magnetic Resonance 1H-NMR (DMSO-d6, ppm): 6.6-6.7(s, hydrogen on phenyl in O-Ar-O); 7.60-7.65(m, hydrogen on phenyl in N-Ar-N); 2.47-2.52(m, hydrogen on methylene in 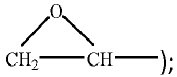 3.00-3.10(m, hydrogen on methyl in 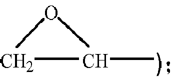 5.05-5.12 (hydrogen on methyl in oxazolidinonyl); 4.03-4.10 (hydrogen on CH₂ connected to epoxy group); 4.18 (hydrogen on CH₂ connected to oxazolidinonyl); 2.35 (hydrogen on methyl).

100 g of epoxy resin C obtained in Example 5, 25.0 g of linear phenolic resin with a phenolic hydroxyl equivalent of 105 g/eq and 0.1 g of 2-phenylimidazole were dissolved in appropriate acetone to form a solution. Standard glass fiber cloth was used for sizing. Then they were pressed into a copper clad laminate, which was named as copper clad laminate e.

The measured properties of the copper clad laminate e are as follows:
dielectric constant(5MHz): 3.5, dielectric loss (5MHz): 0.006, water absorption (%): 0.42, Tg: 168°C, peeling strength: 1.98N.mm⁻¹).

### Example 6

Compound 4 has the following structure:

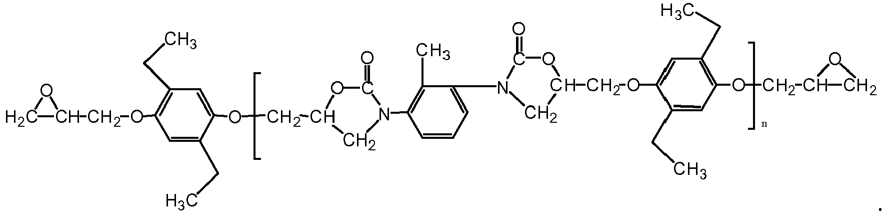

The preparation method is:
(1) 247.5 g (1.5eq) of epoxy resin with an epoxide equivalent of 165 g/eq having the following structure and 0.2 g of 2-methylimidazole were put into a 1000 mL three-neck glass reactor equipped with a stirring apparatus. The mixture was stirred while introducing nitrogen therein, and the temperature was raised to 105°C at the same time. Then 43.5 g of 2,4'-TDI (0.5eq) was dripped evenly taking 240 minutes. After that, the temperature was raised to 130 °C and the mixture continued to react for 640 minutes, and thereby 448.5 g of epoxy resin with epoxide equivalent of 460 g/eq was obtained. The product epoxy resin was named as epoxy resin D.

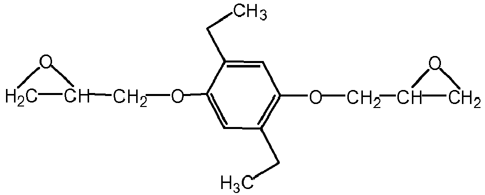

### Structure Characterizations:

Infrared Spectroscopy: epoxy group 913-916cm⁻¹; ether group 1230-1010cm⁻¹; oxazolidonyl group 1755cm⁻¹; methyl 2960 cm⁻¹, 2870 cm⁻¹;

Nuclear Magnetic Resonance 1H-NMR (DMSO-d6, ppm): 6.6-6.7(hydrogen on phenyl in O-Ar-O); 2.30-2.38(hydrogen on phenyl in N-Ar-N); 7.23-7.29(hydrogen on phenyl in N-Ar-N); 7.00-7.06(hydrogen on phenyl in N-Ar-N); 2.47-2.52(hydrogen on methylene in 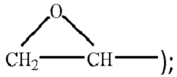 3.00-3.10(hydrogen on methyl in 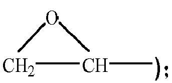 5.05-5.12 (hydrogen on methyl in oxazolidinonyl); 4.03-4.10 (hydrogen on CH₂ connected to epoxy group); 4.18 (hydrogen on CH₂ connected to oxazolidinonyl); 1.24 (hydrogen on methyl); 2.59(hydrogen on methylene).

100 g of epoxy resin D obtained in Example 6, 25.0 g of linear phenolic resin with a phenolic hydroxyl equivalent of 105 g/eq and 0.1 g of 2-phenylimidazole were dissolved in appropriate acetone to form a solution. Standard glass fiber cloth was used for sizing. Then they were pressed into a copper clad laminate, which was named as copper clad laminate f.

The measured properties of the copper clad laminate f are as follows:
dielectric constant(5MHz): 3.4, dielectric loss (5MHz): 0.006, water absorption (%): 0.41, Tg: 170°C, peeling strength: 1.98N.mm⁻¹).

### Example 7

Compound 5 has the following structure:

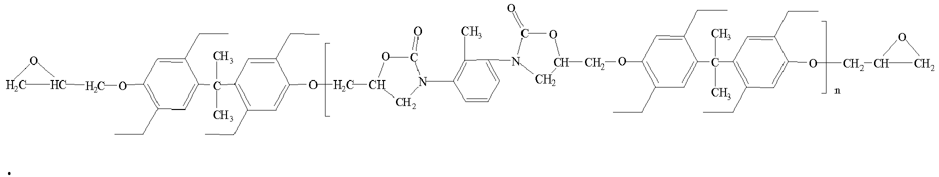

The preparation method is:
(1) 285 g (1.5eq) of epoxy resin with an epoxide equivalent of 190 g/eq having the following structure and 0.1g of tetramethylammonium chloride were put into a 1000 mL three-neck glass reactor equipped with a stirring apparatus. The mixture was stirred while introducing nitrogen therein, and the temperature was raised to 105°C at the same time. Then 26.1 g of 2,4'-TDI (0.3eq) was dripped evenly taking 240 minutes. After that, the temperature was raised to 130 °C and the mixture continued to react for 640 minutes, and thereby 311.1 g of epoxy resin with epoxide equivalent of 259.3 g/eq was obtained. The product epoxy resin was named as epoxy resin E.

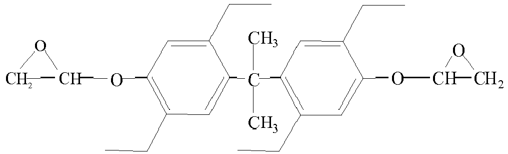

### Structure Characterizations:

Infrared Spectroscopy: epoxy group 913-916cm⁻¹; ether group 1230-1010cm⁻¹; oxazolidonyl group 1755cm⁻¹;

Nuclear Magnetic Resonance 1H-NMR (DMSO-d6, ppm): 1.6-1.7(s, hydrogen on Ar-C(CH₃)₂-Ar); 6.65-6.72(hydrogen on phenyl in O-Ar-C); 7.60-7.65(m, hydrogen on phenyl in N-Ar-N); 2.47-2.52(hydrogen on methylene in 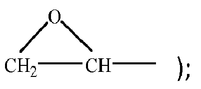 3.00-3.10(hydrogen on methyl in 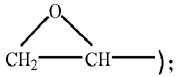 5.05-5.12 (hydrogen on methyl in oxazolidinonyl); 4.03-4.10 (hydrogen on CH₂ connected to epoxy group); 4.18 (hydrogen on CH₂ connected to oxazolidinonyl).

100 g of epoxy resin E obtained in Example 7, 25.0 g of linear phenolic resin with a phenolic hydroxyl equivalent of 105 g/eq and 0.1 g of 2-phenylimidazole were dissolved in appropriate acetone to form a solution. Standard glass fiber cloth was used for sizing. Then they were pressed into a copper clad laminate, which was named as copper clad laminate g.

The measured properties of the copper clad laminate g are as follows:
dielectric constant(5MHz): 3.3, dielectric loss (5MHz): 0.006, water absorption (%): 0.40, Tg: 169°C, peeling strength: 1.98N.mm⁻¹).The applicant states that: the present invention illustrates the technological methods of the present invention by the above examples, but the present invention is not limited to the above technological steps, that is, it does not mean that the present invention must be conducted relying on the above technological steps. Those skilled in the art should understand that any modification to the present invention, any equivalent replacement of each raw material of the present invention and the addition of auxiliary ingredient, the selection of specific embodiment and the like all fall into the protection scope and the disclosure scope of the present invention.

## Claims

1. An isocyanate-modified epoxy resin having a structure of Formula (I): 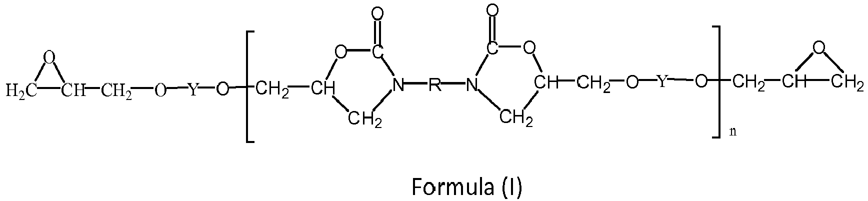 wherein,
R is selected from divalent organic groups;
n is an integer greater than or equal to zero;
Y is selected from 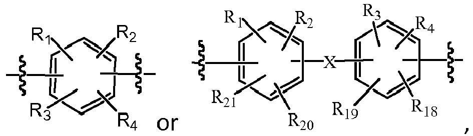 R₁, R₂, R₃, R₄,
R₁₈, R₁₉, R₂₀, R₂₁ are independently selected from organic groups; X is nothingness or is selected from divalent organic groups; and R₁, R₂, R₃, R₄, R₁₈, R₁₉, R₂₀, R₂₁ are not hydrogen atoms at the same time.

2. The epoxy resin of claim 1, **characterized in that**, the epoxy resin has a structure of Formula (II): 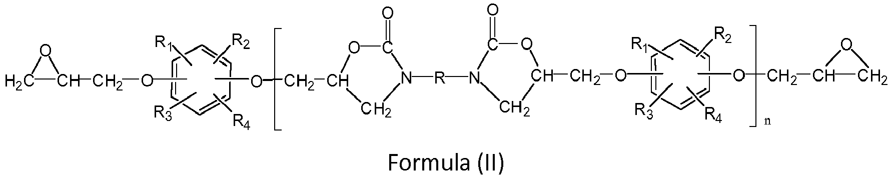 wherein,
R is selected from divalent organic groups;
R₁, R₂, R₃, R₄ are independently selected from organic groups, and R₁, R₂, R₃, R₄ are not hydrogen atoms at the same time;
n is an integer greater than or equal to zero;
or the epoxy resin has a structure of Formula (III): 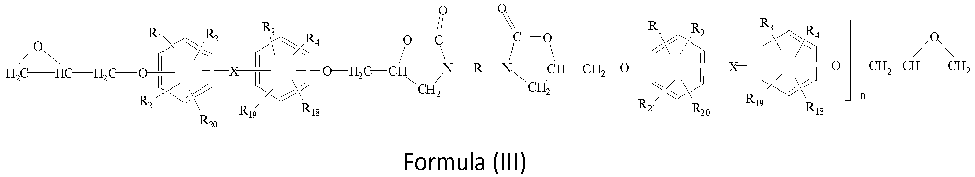 wherein,
X is nothingness or is selected from divalent organic groups;
R is selected from divalent organic groups;
R₁, R₂, R₃, R₄, R₁₈, R₁₉, R₂₀, R₂₁ are independently selected from organic groups, and R₁, R₂, R₃, R₄, R₁₈, R₁₉, R₂₀, R₂₁ are not hydrogen atoms at the same time;
n is an integer greater than or equal to zero.

3. The epoxy resin of claim 1, **characterized in that**, the epoxy resin has a structure of Formula (IV): 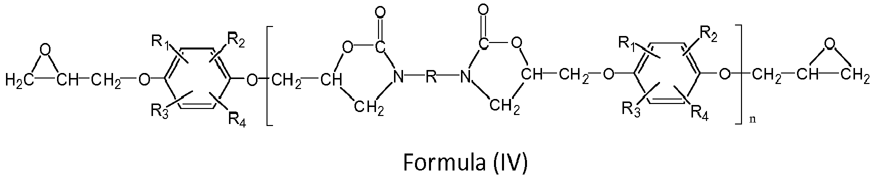 wherein,
R is selected from divalent organic groups;
R₁, R₂, R₃, R₄ are independently selected from organic groups, and R₁, R₂, R₃, R₄ are not hydrogen atoms at the same time;
n is an integer greater than or equal to zero;
or the epoxy resin has a structure of Formula (V): 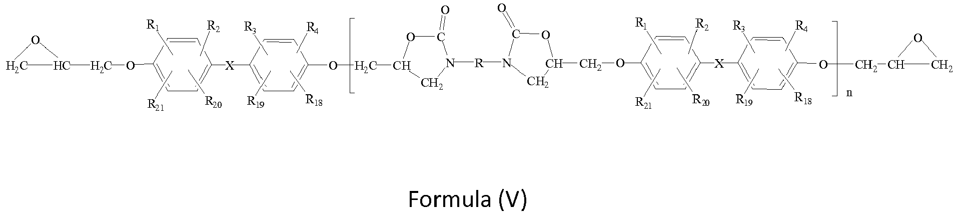 wherein,
X, R are selected from divalent organic groups;
R₁, R₂, R₃, R₄, R₁₈, R₁₉, R₂₀, R₂₁ are independently selected from organic groups, and R₁, R₂, R₃, R₄, R₁₈, R₁₉, R₂₀, R₂₁ are not hydrogen atoms at the same time;
n is an integer greater than or equal to zero.

4. The epoxy resin of any one of claims 1-3, **characterized in that**, R is selected from substituted or unsubstituted straight chain alkylene, substituted or unsubstituted branched alkylene, substituted or unsubstituted arylene/arylidene; preferably C₁-C₃₀ substituted or unsubstituted straight chain alkylene, C₁-C₃₀ substituted or unsubstituted branched alkylene, C₆-C₃₀ substituted or unsubstituted arylene/arylidene; further preferably 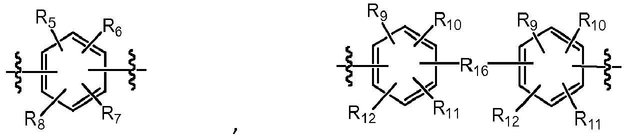 or 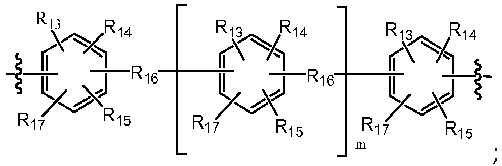 wherein,
R₅, R₆, R₇, R₈, R₉, R₁₀, R₁₁. R₁₂, R₁₃, R₁₄, R₁₅, R₁₇ are independently selected from organic groups, preferably selected from H, substituted or unsubstituted straight chain hydrocarbyl, substituted or unsubstituted branched hydrocarbyl, or substituted or unsubstituted aryl; preferably C₁-C₃₀ substituted or unsubstituted straight chain hydrocarbyl, C₁-C₃₀ substituted or unsubstituted branched hydrocarbyl, or C₆-C₃₀ substituted or unsubstituted aryl;
R₁₆ is independently selected from organic groups, preferably selected from substituted or unsubstituted straight chain alkylene, substituted or unsubstituted branched alkylene, or substituted or unsubstituted arylene/arylidene; preferably C₁-C₃₀ substituted or unsubstituted straight chain alkylene, C₁-C₃₀ substituted or unsubstituted branched alkylene, or C₆-C₃₀ substituted or unsubstituted arylene/arylidene;
m is an integer greater than or equal to zero;
preferably, R₅, R₆, R₇, R₈, R₉, R₁₀, R₁₁. R₁₂ are H;
preferably, R₁₃, R₁₄, R₁₅, R₁₇ are independently selected from substituted or unsubstituted N-oxazolidinonyl;
preferably, R₁₆ is selected from C₁-C₅ substituted or unsubstituted straight chain alkylene, preferably any one of methylene, ethylidene/ethylene or n-propylidene/n-propylene.

5. The epoxy resin of any one of claims 1-3, **characterized in that**, X is nothingness or substituted or unsubstituted straight chain alkylene, substituted or unsubstituted branched alkylene, substituted or unsubstituted arylene/arylidene; preferably nothingness or C₁-C₃₀ substituted or unsubstituted straight chain alkylene, C₁-C₃₀ substituted or unsubstituted branched alkylene, C₆-C₃₀ substituted or unsubstituted arylene/arylidene; further preferably nothingness or any one of methylene, isopropylidene/isopropylene, isobutylidene/isobutylene, cyclohexylidene/cyclohexylene, carbonyl; most preferably nothingness or methylene or isopropylidene/isopropylene; preferably, R is selected from 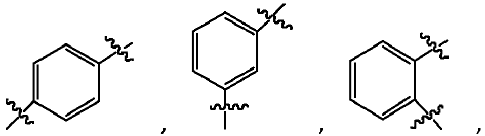 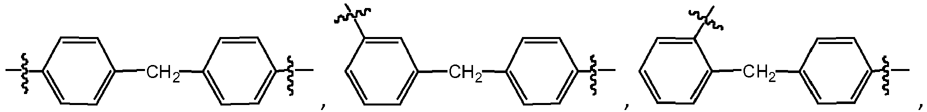 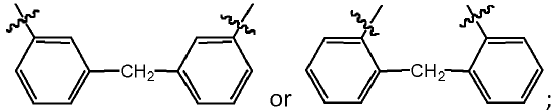
preferably, R₁, R₂, R₃, R₄, R₁₈, R₁₉, R₂₀, R₂₁ are independently selected from H, substituted or unsubstituted straight chain hydrocarbyl, substituted or unsubstituted branched hydrocarbyl, or substituted or unsubstituted aryl; preferably C₁-C₃₀ substituted or unsubstituted straight chain hydrocarbyl, C₁-C₃₀ substituted or unsubstituted branched hydrocarbyl, or C₆-C₃₀ substituted or unsubstituted aryl; preferably any one of methyl, ethyl, n-propyl, isopropyl, n-butyl or tert-butyl, or a combination of at least two of them.

6. An epoxy resin composition, **characterized in that**, the epoxy resin composition comprises an epoxy resin and a curing agent, and a part or all of the epoxy resin is the isocyanate-modified epoxy resin of any one of claims 1-5;
preferably, the epoxy resin also comprises any one of liquid bisphenol A epoxy resin, liquid bisphenol F epoxy resin, solid bisphenol A epoxy resin, solid bisphenol F epoxy resin, bisphenol S epoxy resin, cyclopentadiene epoxy resin or diphenyl epoxy resin, or a combination of at least two of them;
preferably, the curing agent also comprises any one of amines, polyphenol compounds, ester compounds, acids and anhydrides, or a combination of at least two of them;
preferably, the epoxy resin composition also comprises a curing accelerator, and the curing accelerator is any one of imidazole curing accelerators, organophosphine curing accelerators, tertiary amine curing accelerators, or pyridines and derivatives thereof, or a mixture of at least two of them;
preferably, the epoxy resin composition also comprises an inorganic filler; preferably, the epoxy resin composition also comprises a flame retardant; preferably, the epoxy resin composition also comprises a mold discharging agent.

7. A prepreg, **characterized in that**, it is prepared by impregnating a substrate with the epoxy resin composition of claim 6 or coating the epoxy resin composition of claim 6 onto a substrate;
preferably, the substrate is a glass fiber substrate, a polyester substrate, a polyimide substrate, a ceramic substrate or a carbon fiber substrate.

8. A composite metal laminate, **characterized in that**, it comprises more than one sheet of the prepreg of claim 7 and is prepared by coating metal layer on the surface of the prepregs, overlapping and pressing successively;
preferably, the material of the surface-coated metal layer is aluminium, copper, iron and alloys of any combination thereof;
preferably, the composite metal laminate is CEM-1 copper clad laminate, CEM-3 copper clad laminate, FR-4 copper clad laminate, FR-5 copper clad laminate, CEM-1 aluminum clad laminate, CEM-3 aluminum clad laminate, FR-4 aluminum clad laminate or FR-5 aluminum clad laminate.

9. A wiring board, **characterized in that**, it is prepared by processing wires on the surface of the composite metal laminate of claim 8.
